# EUROPEAN PATENT SPECIFICATION

(11) **EP 1 698 614 B1**
(45) Date of publication and mention of the grant of the patent: **13.07.2011**
(21) Application number: 04820870.6
(22) Date of filing: 09.11.2004
(51) Int. Cl.: C07F 7/00, C23C 16/18, C23C 16/40

(54) **METAL COMPOUND, MATERIAL FOR FORMING THIN FILM AND METHOD FOR PREPARING THIN FILM**
METALLVERBINDUNG, MATERIAL ZUR AUSBILDUNG EINES DÜNNEN FILMES UND VERFAHREN ZUR HERSTELLUNG EINES DÜNNEN FILMES
COMPOSE METALLIQUE, MATERIAU PERMETTANT DE FORMER UN FILM MINCE, ET PROCEDE DE PREPARATION D'UN FILM MINCE

(30) Priority: 25.12.2003 JP 2003428981
(43) Date of publication of application: 06.09.2006
(73) Proprietor: Adeka Corporation, Tokyo 116-8554 (JP)
(72) Inventor: SAKURAI, Atsushi;, e, Arakawa-ku, Tokyo 1160012; (JP); YAMADA, Naoki;, e, Arakawa-ku, Tokyo 1160012; (JP)
(74) Representative: Forstmeyer, Dietmar
(86) International application number: PCT/JP2004/016579
(87) International publication number: WO 2005/063685

(56) References cited:
- WO-A-03/035926
- WO-A1-93/18202
- WO-A1-03/040150
- JP-A- 2000 351 784
- US-B1- 6 274 195
- LEE, J.-H. ET AL: "Effect of the precursors on the deposition of (Ba, Sr)TiO3 films" JOURNAL DE PHYSIQUE IV: PROCEEDINGS , 11(PR3, THIRTEENTH EUROPEAN CONFERENCE ON CHEMICAL VAPOR DEPOSITION, 2001), PR3/215-PR3/222 CODEN: JPICEI; ISSN: 1155-4339, 2001, XP008070510
- BHARARA ET AL: 'Reactions of Titanium Alkoxides with N-Methylaminoalcohols' Z.ANORG.ALLG.CHEM. vol. 403, no. 3, 1974, pages 337 - 346, XP002986956

## Description

### Technical Field

This invention relates to a novel metal compound (lead compound, titanium compound and zirconium compound) having a specific amino alcohol as a ligand, a material for thin film formation containing the metal compound, and a process for forming a metal-containing thin film using the material.

### Background Art

A thin film containing lead, titanium or zirconium is chiefly used as a member of electronic components, such as high dielectric constant capacitors, ferroelectric capacitors, gate insulators, and barrier films.

Processes for forming the above-described thin film include flame hydrolysis deposition, sputtering, ion plating, MOD techniques including dipping-pyrolysis process and sol-gel process, and chemical vapor deposition (hereinafter sometimes abbreviated as CVD). Chemical vapor deposition processes inclusive of ALD (atomic layer deposition) are the most suitable for many advantages, such as compositional controllability, excellent step coverage, suitability to large volume production, and capability of hybrid integration.

MOD and CVD processes use a metal compound having an organic ligand as a precursor supplying a metal to a thin film. For example, bis(dipivaroylmethanato)lead having dipivaroylmethane as a ligand is employed as a lead compound, but it does not have sufficient volatility. It has been reported that a titanium compound or a zirconium compound having, as a ligand, an alcohol terminated with an ether group or an amino group that is a donor group capable of coordinating to a metal atom has a relatively high vapor pressure and offers conditions for stable thin film formation. For example, Patent Document: land Patent Document:2 disclose a metal compound using an ether alcohol, and Patent Document:3 discloses a metal compound using an amino alcohol.

Non-Patent Document: 1 reports a lead compound using an ether alcohol and an amino alcohol as a ligand.

Patent Document: 1 JP-A-6-321824
Patent Document:2 JP-A-2002-69641
Patent Document:3 JP-A-2000-351784
Non-Patent Document:1 Inorganic Chemistry, vol. 29, No. 23, 1990, pp. 4640-4646

### Disclosure of the Invention

Metal compounds suitable as a material in thin film formation processes involving vaporization of the metal compound, such as CVD, are required to have a low melting point and therefore be deliverable in a liquid state and to have a high vapor pressure and therefore be easy to vaporize. Where two or more metal compounds are mixed and used as a metal compound composition, each metal compound is required not to undergo denaturation by ligand exchange or any chemical reaction when mixed up or while stored. However, there is no metal compound of lead, titanium or zirconium that satisfies these requirements.

As a result of extensive investigations, the present inventors have found that the above problem is solved by a specific metal compound endowed with steric hindrance effect of a tertiary alkoxide and thus reached the present invention.

The present invention provides a metal compound represented by general formula (I) shown below, a material for thin film formation containing the metal compound, and a process of forming a metal-containing thin film using the material.

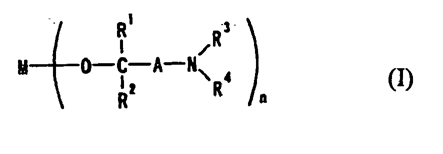

wherein R¹, R², R³, and R⁴ each represent an alkyl group having 1 to 4 carbon atoms; A represents an alkanediyl group having 1 to 8 carbon atoms; M represents a lead atom, a titanium atom or a zirconium atom; n represents 2 when M is a lead atom or 4 when M is a titanium or zirconium atom.

### Brief Description of the Drawing

Fig. 1 is a schematic diagram illustrating an example of a CVD system that can be used to carry out the thin film formation process according to the invention.
Fig. 2 is a schematic diagram illustrating another example of a CVD system that can be used to carry out the thin film formation process according to the invention.
Fig. 3 is a schematic diagram illustrating still another example of a CVD system that can be used to carry out the thin film formation process according to the invention.

### Best Mode for Carrying out the Invention

The metal compound of the invention is represented by general formula (I) and is suitable as a precursor in thin film formation processes involving vaporization, such as CVD and ALD. The metal compound of the invention has a dialkylamino group with strong donor effect and large steric hindrance introduced to the terminal of its ligand. Steric hindrance is also introduced to near the oxygen atom as a tertiary alcohol thereby to reduce and/or block the electrical polarity between the metal atom and the oxygen atom. As a result, the metal compound is suppressed from molecular association and thus endowed with high volatility and prevented from unnecessary chemical reactions.

In general formula (I), the alkyl group having 1 to 4 carbon atoms as represented by R¹, R², R³, and R⁴ includes methyl, ethyl, propyl, isopropyl, butyl, secbutyl, tert-butyl, and isobutyl. The alkanediyl group as represented by A may have a straight-chain configuration or may have one or more branches at any position(s) as long as the total carbon atom number is from 1 to 8. The alkanediyl group is preferably the one which makes an energetically stable 5- or 6-membered ring when the terminal donor group, the dialkylamino group, is coordinated to the metal atom. Such a preferred alkanediyl group includes a group represented by general formula (II) shown below. The metal compound of the invention can include optical isomers but is not distinguished by the isomeric configuration.

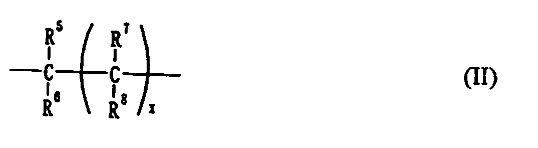

wherein R⁵, R⁶, R⁷, and R⁸ each represent a hydrogen atom or an alkyl group having 1 to 4 carbon atoms; and x represents 0 or 1; provided that the total number of carbon atoms in R⁵, R⁶, R⁷, and R⁸ is 1 to 8.

The compound in which the terminal donor group of a ligand is coordinated to the metal atom to form a cyclic structure is represented by general formula (III) below. The metal compound of the invention represented by general formula (I) is not distinguished from the metal compound represented by general formula (III). That is, the metal compound of general formula (I) includes in its scope the compound of general formula (III).

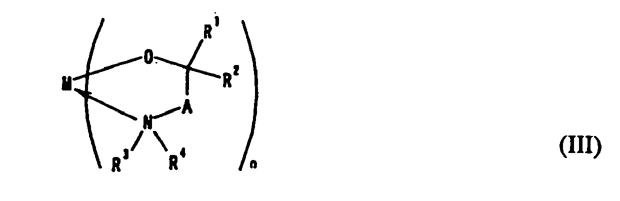

wherein R¹, R², R³, and R⁴ each represent an alkyl group having 1 to 4 carbon atoms; A represents an alkanediyl group having 1 to 8 carbon atoms; M represents a lead atom, a titanium atom or a zirconium atom; n represents 2 when M is a lead atom or 4 when M is a titanium atom or a zirconium atom.

Specific examples of the metal compound of the invention include compound Nos. 1 through 36 listed below.

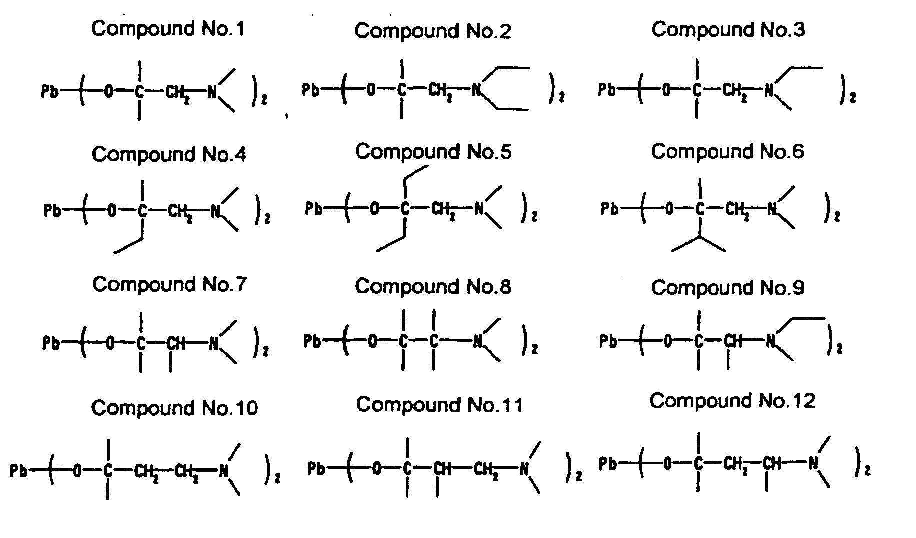

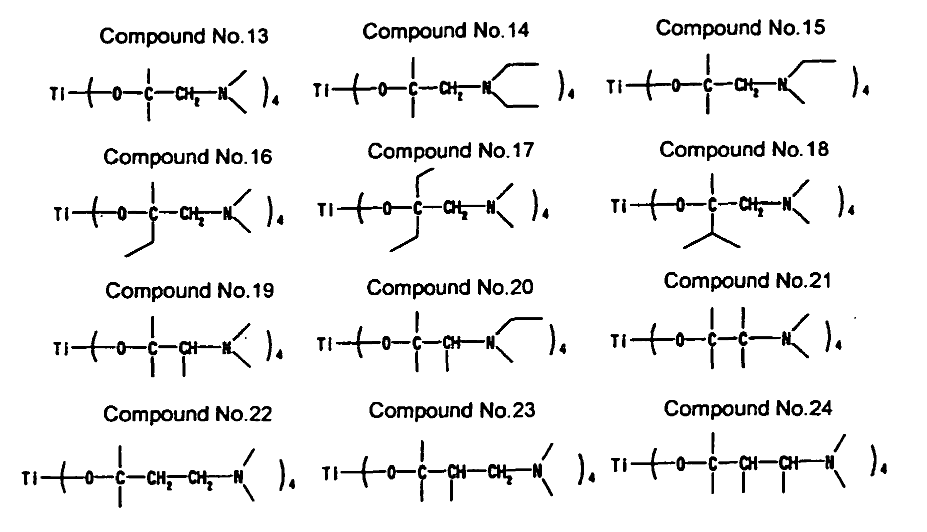

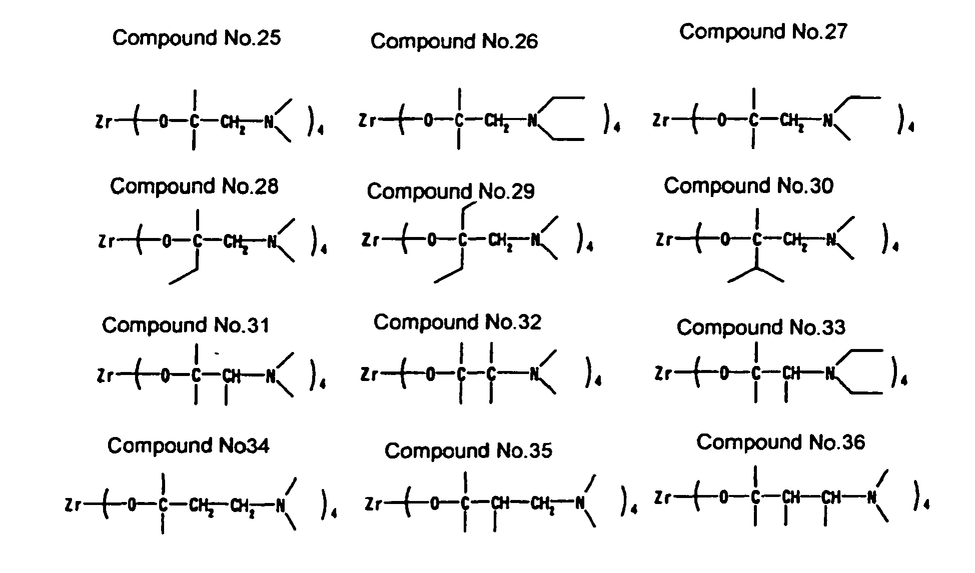

Where the metal compound of the invention is used as a material of thin film formation involving the step of vaporization, it is preferred that R¹ to R⁴ and A in the above general formula (I) have a smaller molecular weight to have a higher vapor pressure. Specifically, R¹ to R⁴ are each preferably a methyl group, and A is preferably a methylene group. Where the metal compound of the invention is used as a material of thin film formation by MOD involving no vaporization step, R¹ to R⁴ and A are selected arbitrarily according to solubility in the solvent used and reactivity in thin film formation.

The metal compound of the invention is not limited by the process of preparation and can be prepared by using well-known reactions. Widely known processes for synthesizing general metal alkoxides can be applied using a corresponding tertiary amino alcohol. Such processes include a process comprising reacting a halide or an inorganic salt (e.g., a nitrate) or its hydrate of a metal with a corresponding alcohol compound in the presence of a base such as sodium, sodium hydride, sodium amide, sodium hydroxide, sodium methylate, ammonia or amine; a process comprising reacting a halide or an inorganic salt (e.g., a nitrate) or its hydrate of a metal with an alkali metal alkoxide (e.g., sodium alkoxide, lithium alkoxide or potassium alkoxide) of a corresponding alcohol compound; a process comprising an alcohol exchange reaction between a metal alkoxide of a low-molecular alcohol, such as a metal methoxide, ethoxide, isopropoxide or butoxide, and a corresponding alcohol compound; and a process comprising reacting a metal halide or inorganic salt (e.g., nitrate) with a derivative providing a reactive intermediate and reacting the intermediate with an alcohol compound.

The metal compound of general formula (I) in which M is lead can be obtained by reacting lead chloride with bis(trimethylsilyl)aminolithium to synthesize a reactive intermediate, bis(bis(trimethylsilyl)amino)lead, which is then reacted with a tertiary amino alcohol. The metal compound of general formula (I) in which M is titanium or zirconium can be obtained through an alcohol exchange reaction between a metal alkoxide and a tertiary amino alcohol.

The material for thin film formation according to the invention contains the aforementioned metal compound of the invention as a thin film precursor. The form of the material depends on the thin film formation technique using the material, including MOD processes, such as dipping-pyrolysis and sol-gel process, and CVD processes inclusive of ALD. The metal compound of the invention is especially useful as a raw material of CVD in view of its physical properties.

Where the material for thin film formation of the invention is for chemical vapor deposition (CVD), the form of the material is selected as appropriate to the procedures of the CVD process adopted, such as a source delivery system.

The source delivery system includes a vapor delivery system in which the material for CVD is vaporized by heating and/or pressure reduction in a container and introduced into a deposition reaction site, if desired, together with a carrier gas, e.g., argon, nitrogen or helium, and a liquid delivery system in which the material for CVD is delivered in the form of a liquid or a solution to a vaporizer, where it is vaporized by heating and/or pressure reduction and then led to a deposition reaction site. When a plurality of materials for thin film formation are used in a liquid delivery system, as many vaporizers as the materials may be provided, and the vapor from each vaporizer is separately introduced to the deposition reaction site. Alternatively, the materials may be introduced into a vaporizer, and the resulting mixed vapor is delivered to the deposition reaction site. When applied to the vapor delivery system, the metal compound represented by general formula (I) of the invention *per se* is a material for CVD. In the case of the liquid delivery system, the metal compound represented by general formula (I) of the invention *per se* or a solution of the metal compound in an organic solvent is a material for CVD.

In a multi-component CVD process, the source delivery systems includes a system in which a plurality of the materials are separately vaporized and delivered (hereinafter referred to as a multi-source system) and a system in which a plurality of the materials are previously mixed at a prescribed ratio, and the mixture is vaporized and delivered (hereinafter referred to as a single source system). In the case of the single source system, the material for CVD is a mixture or mixed solution of the metal compounds of the invention or a mixture or mixed solution of the metal compound(s) of the invention and other precursor(s). For example, a mixture or mixed solution containing at least one of compound Nos. 1 to 12, at least one of compound Nos. 13 to 24, and at least one of compound Nos. 25 to 36 is a preferred single source for PZT (lead zirconate titanate), which is a ferroelectric substance. A mixture or mixed solution of the metal compound of general formula (I) in which M is lead, tetrakis(1-methoxy-2-methyl-2-propoxy)titanium, and tetrakis(1-methoxy-2-methyl-2-propoxy)zirconium is also preferred as a single source for PZT.

The organic solvent that can be used in the material for CVD is not particularly limited, and any widely known organic solvent is useful. Examples are alcohols, such as methanol, ethanol, 2-propanol, and n-butanol; acetic esters, such as ethyl acetate, butyl acetate, and methoxyethyl acetate; ether alcohols, such as ethylene glycol monomethyl ether, ethylene glycol monoethyl ether, ethylene glycol monobutyl ether, and diethylene glycol monomethyl ether; ethers, such as tetrahydrofuran, tetrahydropyran, ethylene glycol dimethyl ether, diethylene glycol dimethyl ether, triethylene glycol dimethyl ether, and dibutyl ether; ketones, such as methyl butyl ketone, methyl isobutyl ketone, ethyl butyl ketone, dipropyl ketone, diisobutyl ketone, methyl amyl ketone, cyclohexanone, and methylcyclohexanone; hydrocarbons, such as hexane, cyclohexane, methylcyclohexane, dimethylcyclohexane, ethylcyclohexane, heptane, octane, toluene, and xylene; hydrocarbons having a cyano group, such as 1-cyanopropane, 1-cyanobutane, 1-cyanohexane, cyanocyclohexane, cyanobenzene, 1,3-dicyanopropane, 1,4-dicyanobutane, 1,6-dicyanohexane, 1,4-dicyanocyclohexane, and 1,4-dicyanobenzene; pyridine, and lutidine. A solvent or a mixture of solvents to be used is selected according to, for example, solubility for the solute and the boiling temperature or ignition temperature in relation to the working temperature. In using these organic solvents, the total concentration of the metal compound(s) of the invention and other precursor(s) in the organic solvent is preferably 0.01 to 2.0 mol/l, still preferably 0.05 to 1.0 mol/l.

Other precursors that can be used in combination with the metal compound of the invention in the multi-component CVD system are not particularly limited, and any precursors well-known in the art for use as CVD materials can be used.

The other precursors include compounds formed between a metal and one or more organic coordinating compounds selected from alcohol compounds, glycol compounds, β-diketones, cyclopentadiene compounds, and so forth. The metal species include magnesium, calcium, strontium, barium, titanium, zirconium, hafnium, vanadium, niobium, tantalum, manganese, iron, ruthenium, cobalt, rhodium, iridium, nickel, palladium, platinum, copper, silver, gold, zinc, gallium, indium, germanium, tin, lead, antimony, bismuth, silicon, yttrium, lanthanum, cerium, praseodymium, neodymium, promethium, samarium, europium, gadolinium, terbium, dysprosium, holmium, erbium, thulium, and ytterbium.

If desired, the material for CVD can contain a nucleophilic reagent to stabilize the metal compound of the invention and other precursor. Examples of the nucleophilic reagent include ethylene glycol ethers, such as glyme, diglyme, triglyme, and tetraglyme; crown ethers, such as 18-crown-6, dicyclohexyl-18-crown-6, 24-crown-8, dicyclohexyl-24-crown-8, and dibenzo-24-crown-8; polyamines, such as ethylenediamine, N,N'-tetramethylethylenediamine, diethylenetriamine, triethylenetetramine, tetraethylenepentamine, pentaethylenehexamine, 1,1,4,7,7-pentamethyldiethylenetriamine, and 1,1,4,7,10,10-hexamethyl-triethylenetetramine; cyclic polyamines, such as cyclam and cyclen; β-keto esters, such as methyl acetoacetate, ethyl acetoacetate, and 2-methoxyethyl acetoacetate; and β-diketones, such as acetylacetone, 2,4-hexanedione, 2,4-heptanedione, 3,5-heptanedione, and dipivaroylmethane. The nucleophilic reagent as a stabilizer is used in an amount of 0.1 to 10 mol, preferably 1 to 4 mol, per mole of the precursor.

The process of forming a thin film according to the present invention is by CVD using the metal compound of the invention and, if necessary, other precursor. CVD is a process in which a vaporized material and, if necessary, a reactive gas is/are led to a substrate and allowed to decompose and/or chemically react on the substrate, and a thin film is allowed to grow and build up on the substrate. The process of the present invention is not particularly restricted by the method of material delivery, the mode of deposition, the film formation conditions, the film formation equipment, and the like. Any conditions and methods commonly known in the art are made use of.

The reactive gas which can be used if necessary includes oxidizing gases, such as oxygen, ozone, nitrogen dioxide, nitrogen monoxide, water vapor, hydrogen peroxide, formic acid, acetic acid, and acetic anhydride; and reducing gases, such as hydrogen. Reactive gases that can be used to form a nitride film include organic amine compounds, such as monoalkylamines, dialkylamines, trialkylamines, and alkylenediamines; hydrazine; and ammonia.

The method of material delivery includes the above-described vapor delivery system, liquid delivery system, single source system, and multi-source system.

In the case of a multi-source system, all the materials may the metal compounds of the invention, or the materials may be a combination of the metal compound(s) of the invention with other precursors. Where the metal compound of the invention is used in combination with other precursor, it is preferred that the metal compound of the invention and the other precursor to be combined exhibit similar behavior in decomposition associated with a thin film forming reaction. For instance, a combination of the metal compound of the invention as a lead precursor and a tetraalkoxide as a titanium precursor or a zirconium precursor is preferred. Preferred examples of the tetraalkoxide as a titanium precursor include tetrakis(1-methoxy-2-methyl-2-propoxy)titanium and tetra(tert-butoxy)titanium. Preferred examples of the tetraalkoxide as a zirconium precursor include tetrakis(1-methoxy-2-methyl-2-propoxy) and tetra(tert-butoxy)zirconium. In using compound No. 1 as a lead precursor, preferred titanium precursors to be combined include tetra(ethoxy)titanium, tetra(2-propoxy)titanium, tetra(butoxy)titanium, tetra(tert-butoxy)titanium, tetra(tert-amyl)titanium, and tetrakis(1-methoxy-2-methyl-2-propoxy)titanium, and preferred zirconium precursors to be combined include tetra(ethoxy)zirconium, tetra(2-propoxy)zirconium, tetra(butoxy)zirconium, tetra(tert-butoxy)zirconium, tetra(tert-amyl)zirconium, and tetrakis(1-methoxy-2-methyl-2-propoxy)zirconium.
In the case of a multi-source system, the material for thin film formation containing the other precursor may be either the precursor itself or a solution of the precursor in the above-described organic solvent. The concentration of the other precursor in the organic solvent is preferably 0.01 to 2.0 mol/l, still preferably 0.05 to 1.0 mol/l. The material containing the other precursor may contain the above-described nucleophilic reagent in an amount of 0.1 to 10 mol, preferably 1 to 4 mol, per mole of the other precursor.

The deposition modes include thermal CVD (only heat is used to cause the vaporized material or a mixture of the vaporized material and a reactive gas to react to deposit a film), plasma-enhanced CVD (heat and plasma are used), photo-assisted CVD (heat and light are used), photo plasma-assisted CVD (heat, light, and plasma are used), and atomic layer deposition (ALD) in which a deposition reaction of CVD is divided into elementary reaction steps so as to build up a film stepwise on a molecular level.

The film formation conditions include reaction temperature (the substrate temperature), reaction pressure, and deposition rate. The reaction temperature is preferably 160°C or higher at which the metal compound of the invention reacts sufficiently, still preferably 250° to 800°C. The reaction pressure is from atmospheric pressure to 10 Pa for thermal CVD and photo-assisted CVD or from 10 to 2000 Pa for film formation using plasma. The deposition rate can be controlled by the material feed conditions (vaporizing temperature and vaporizing pressure) and the reaction temperature and pressure. Too high a deposition rate tends to result in deteriorated characteristics of the resulting thin film, and too low a deposition rate can result in poor productivity. A preferred deposition rate ranges from 0.5 to 5000 nm/min, still preferably 1 to 1000 nm/min. In the case of ALD, the film thickness is controlled by the number of cycles to reach a desired film thickness.

In the film formation process of the present invention, the resulting thin film may be subjected to annealing to obtain improved electrical characteristics. Where step coverage is required, the process can have the step of reflowing the thin film. The reflow is conducted at 500° to 1200°C, preferably 600° to 800°C.

Combined with appropriate selections of a precursor of other component, a reactive gas, and film forming conditions, the material for thin film formation according to the invention and the process of thin film formation according to the invention provide a thin film of desired kind, such as oxide ceramics, nitride ceramics, and glass. The thin films produced in the invention include an oxide thin film of lead, titanium or zirconium oxide; a double or complex oxide thin film of lead titanate, lead zirconate, lead zirconate titanate, rare earth element-doped lead zirconate titanate, bismuth titanate, rare earth element-doped bismuth titanate, strontium titanate, barium titanate, strontium barium titanate, a titanium-doped silicate, a zirconium-doped silicate, zirconium-doped alumina, yttrium-stabilized zirconia, a rare earth element-zirconium double oxide, a rare earth element-silicon-zirconium complex oxide or a titanium-tantalum double oxide; titanium nitride, and zirconium nitride. Applications of the thin films of oxide ceramics include high dielectric constant capacitor films, gate insulators, ferroelectric capacitor films, and capacitor films. Applications of the thin films of nitride ceramics include barrier layers. Applications of the glass thin films include optical glass applications, such as optical fibers, optical waveguides, optical amplifiers, and optical switches.

### Examples:

The present invention will now be illustrated in greater detail with reference to Examples and Evaluation Examples, but it should be understood that the invention is not construed as being limited thereto.

### EXAMPLE 1: Preparation of compound No. 1

In a light-protected flask were put 0.478 mol of lead dichloride, 2000 ml of dehydrated diethyl ether, and 0.935 mol of bis(trimethylsilyl)aminolithium and stirred at 25°C for 24 hours in a dry argon atmosphere. The diethyl ether solvent was removed by evaporation, and 3000 ml of dehydrated hexane was added to the residue. The solid phase was removed by filtration. To the resulting solution was added dropwise 1.03 mol of 1-dimethylamino-2-methyl-2-propanol, followed by stirring at 25°C for 24 hours. Hexane and by-produced hexamethyldisilazane were removed by distillation. From the fraction at 13 to 15 Pa and a tower top temperature of 90° to 95°C was obtain white crystals in a yield of 38%, which were further purified by distillation under reduced pressure to obtain crystals. The recovery of the purification was 90%. The resulting crystals were identified to be the title compound, compound No. 1. The analytical values of the crystals were as follows.

### Results of analyses:

(1) Elemental analysis (metal analysis: ICP-AES)
   Pb: 48.1 % by mass (calcd.: 47.1 %)
(2) ¹H-NMR (solvent: deuterobenzene) (chemical shift: multiplicity:number of hydrogens)
   (1.45:s:12) (2.26a:12) (2.56:s:4)
(3) TG-DTA (Ar: 100 ml/min; temperature rise: 10°C/min; amount of sample: 9.665 mg)
   Melting point: 61°C
   50% mass loss temperature: 191°C

### EXAMPLE 2: Preparation of compound No. 13

Into a reaction flask were dropped 0.100 mol of tetra(2-propoxy)titanium, 60 ml of dehydrated xylene, and 0.440 mol of 1-dimethylamino-2-methyl-2-propanol and allowed to react at 135°C for 18 hours in a dry argon atmosphere while removing by-produced 2-propanol by evaporation. Xylene was removed by evaporation, and the residue was distilled under reduced pressure. A pale yellow liquid was obtained from the fraction at 5 to 7 Pa and a tower top temperature of 122° to 124°C in a yield of 48%. The pale yellow liquid was purified by distillation under reduced pressure to obtain a liquid. The recovery of the purification was 94%. The resulting liquid was identified to be the title compound, compound No. 13. The results of analyses on the liquid are shown below.

### Results of analyses:

(1) Elemental analysis (metal analysis: ICP-AES)
   Ti: 9.43% by mass (calcd.: 9.34%)
(2) ¹H-NMR (solvent: deuterobenzene) (chemical shift: multiplicity:number of hydrogens)
   (1.50:s:24) (2.36:s:24) (2.48:s:8)
(3) TG-DTA (Ar: 100 ml/min; temperature rise: 10°C/min; amount of sample: 6.175 mg)
   50% mass loss temperature: 239°C

### EXAMPLE 3: Preparation of compound No. 25

Into a reaction flask were dropped 0.100 mol of Zirconium(IV) isopropoxide isopropanol complex, 60 ml of dehydrated xylene, and 0.440 mol of 1-dimethylamino-2-methyl-2-propanol and allowed to react at 135°C for 10 hours in a dry argon atmosphere while removing by-produced 2-propanol by evaporation. Xylene was removed by evaporation, and the residue was distilled under reduced pressure. A colorless liquid was obtained from the fraction at 8 to 10 Pa and a tower top temperature of 139° to 140°C in a yield of 53%. The colorless liquid was purified by distillation under reduced pressure to obtain a liquid. The recovery of the purification was 92%. The resulting liquid was identified to be the title compound, compound No. 25. The results of analyses on the liquid are shown below.

### Results of analyses:

(1) Elemental analysis (metal analysis: ICP-AES)
   Zr: 16.8% by mass (calcd.: 16.4%)
(2) ¹H-NMR (solvent: deuterobenzene) (chemical shift: multiplicity:number of hydrogens)
   (1.45:s:24) (2.37:s:24) (2.53:s:8)
(3) TG-DTA (Ar: 100 ml/min; temperature rise: 10°C/min; amount of sample: 7.975 mg)
   50% mass loss temperature: 242°C

### EVALUATION EXAMPLE 1: Evaluation on volatility of lead compounds

The thermal behavior of comparative compounds 1 and 2 shown below was observed by TG-DTA under the same conditions as in Example 1. In table 1 are shown the results obtained as well as the thermal behavior of compound No. 1.

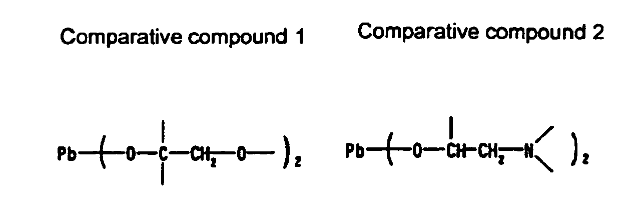

**TABLE 1**

| Metal Compound | 50% Mass Loss Temperature | Residue at 300°C | Remark |
|---|---|---|---|
| Compound No.1 | 191°C | 1.0% | mass loss due to volatilization |
| Comparative Compound 1 | 334°C | 65.2% | mass loss due to volatilization and decomposition |
| Comparative Compound 2 | 211°C | 28.0% | mass loss due to volatilization and decomposition |

It is seen from Table 1 that compound No. 1, which has the largest molecular weight of the three metal compounds tested, is superior in volatility to the comparative compounds. Compound No. 1 was thus proved more suitable as a lead precursor for CVD than comparative compounds 1 and 2.

### EVALUATION EXAMPLE 2: Evaluation on volatility of titanium compounds and zirconium compounds

The thermal behavior of comparative compounds 3 and 4 shown below was observed by TG-DTA under the same conditions as in Examples 2 and 3. In table 2 are shown the results obtained as well as the thermal behavior of compound Nos. 13 and 25.

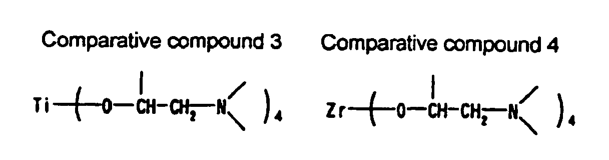

**TABLE 2**

| Metal Compound | 50% Mass Loss Temperature | Residue at 300°C | Remark |
|---|---|---|---|
| Compound No. 13 | 239°C | 0.6% | mass loss due to volatilization |
| Compound No. 25 | 242°C | 08% | mass loss due to volatilization |
| Comparative Compound 3 | 256°C | 1.0% | mass loss due to volatilization |
| Comparative Compound 4 | 290°C | 1.0% | mass loss due to volatilization |

It is seen from Table 2 that compound Nos. 13 and 25, which have a larger molecular weight than the respectively corresponding comparative compounds, are superior in volatility to the comparative compounds. Compound Nos. 13 and 25 were thus proved more suitable as a precursor for CVD than the comparative compounds.

### EVALUATION EXAMPLE 3: Evaluation on composition stability

A composition prepared by mixing compound No. 1, compound No. 13, and compound No. 25 at a molar ratio of 1:1:1 was analyzed by TG-DTA immediately after mixing and after the composition was stored at 100°C for 24 hours. TG-DTA was carried out under the same conditions as in Example 1.
As a result, the composition immediately after mixing showed mass loss due to volatilization. The 50% mass loss temperature was 230°C, and the residue at 300°C was 1.5%. The composition after 100°C, 24 hour storage also showed mass loss by volatilization. The 50% mass loss temperature was 230°C, and the residue at 300°C was 1.6%. These results provided confirmation that the mixture had undergone no denaturation.

### EXAMPLE 4: Production of PZT thin film

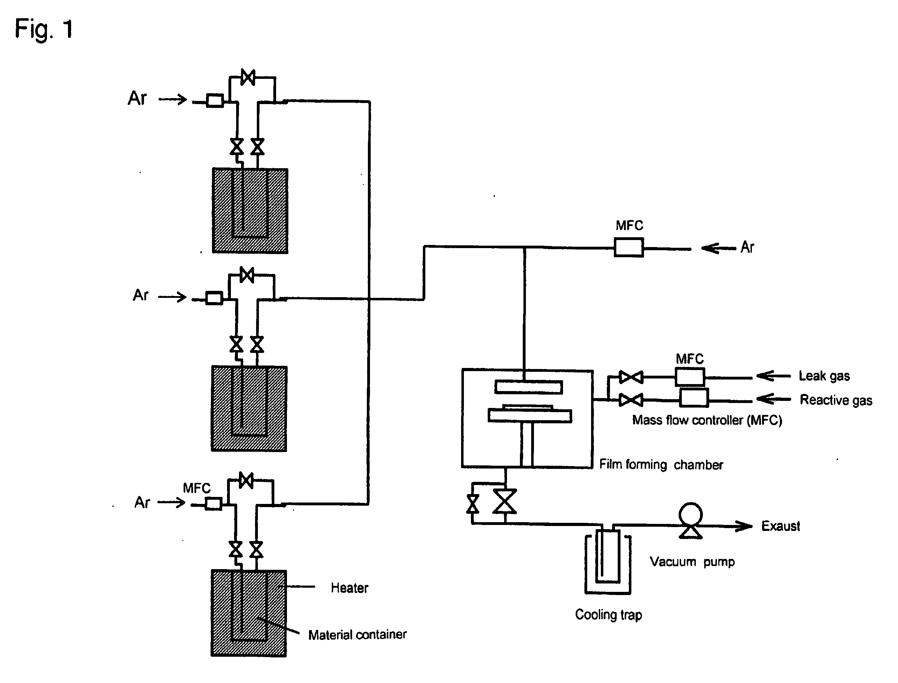

A PZT thin film was formed on a silicon wafer using the CVD system shown in Fig. 1 under the following conditions. The thickness and composition of the resulting film were measured by X-ray fluorescence analysis. The results of measurement are shown below.

### Film formation conditions:

Lead source: compound No. 1 (material temperature: 170°C; pressure:
1300 Pa; carrier gas: argon 200 sccm)
Titanium source: compound No. 13 (material temperature: 182°C; pressure: 1300 Pa; carrier gas: argon 200 sccm)
Zirconium source: compound No. 25 (material temperature: 175°C; pressure:
1300 Pa: carrier gas: argon 200 sccm)
Oxidizing gas: oxygen 300 sccm
Reaction pressure: 3000 Pa
Reaction temperature (substrate temperature): 400°C
Film forming time: 25 min

### Results:

Film thickness: 130 nm
Composition (by mole): Pb/Ti/Zr= 1.00/0.52/0.47

### EXAMPLE 5: Formation of PZT thin film

A PZT thin film was formed on a silicon wafer using the CVD system shown in Fig. 1 under the following conditions. The thickness and composition of the resulting film were measured by X-ray fluorescence analysis. The results of measurement are shown below.

### Film formation conditions:

Lead source: compound No. 1 (material temperature: 170°C; pressure: 1300 Pa; carrier gas: argon 200 sccm)
Titanium source: tetra(tert-butoxy)titanium (material temperature: 125°C;
pressure: 1300 Pa; carrier gas: argon 200 sccm)
Zirconium source: tetra(tert-butoxy)zirconium (material temperature: 125°C;
pressure: 1300 Pa: carrier gas: argon 200 sccm)
Oxidizing gas: oxygen 300 sccm
Reaction pressure: 3000 Pa
Reaction temperature (substrate temperature): 400°C
Film forming time: 20 min

### Results:

Film thickness: 130 nm
Composition (by mole): Pb/Ti/Zr=1.00/0.52/0.58

### EXAMPLE 6: Formation of PZT thin film

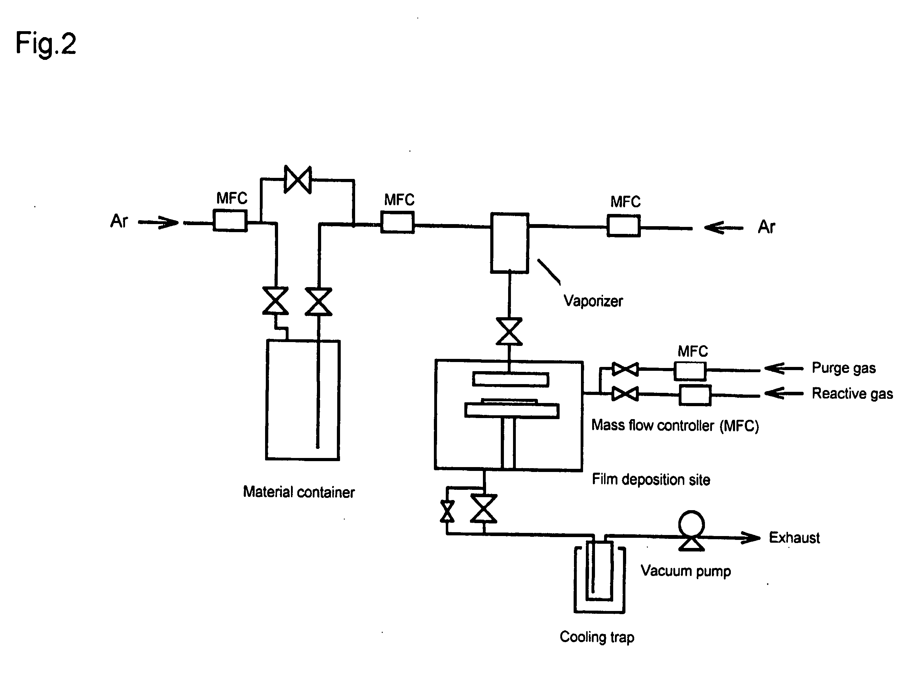

To 500 ml of ethylcyclohexane dehydrated to a water content less than 1 ppm were added 0.1 mol of compound No. 1, 0.05 mol of compound No. 13, and 0.05 mol of compound No. 25 to prepare a material for CVD. A thin film was formed using the CVD system shown in Fig. 2 and the resulting material for CVD in accordance with the following conditions and steps. The thickness and composition of the resulting film were measured in the same manner as in Example 4. The results of measurement are shown below.

### Conditions:

Material flow rate: 1.20 sccm
Reaction temperature (substrate temperature): 300°C
Reactive gas: water vapor

### Steps:

A series of steps (1) through (4) below, which make one cycle, were repeated 300 times (cycles). Finally, the film formed was annealed at 600°C for 3 minutes.
   (1) The CVD material vaporized under conditions of a vaporizer temperature of 170°C and a vaporizer pressure of 1300 to 1400 Pa was introduced and deposited under a system pressure of 1300 to 1400 Pa for 1 second.
   (2) The atmosphere was purged with argon for 2 seconds to expel any unreacted material.
   (3) Water vapor was introduced to cause reaction under 1300 Pa for 1 second.
   (4) The atmosphere was purged with argon for 2 seconds to remove any unreacted material.

### Results:

Film thickness: 85 nm
Composition (by mole): Pb/TT/Zr=1.00/0.56/0.49

### EXAMPLE 7: Formation of PZT film

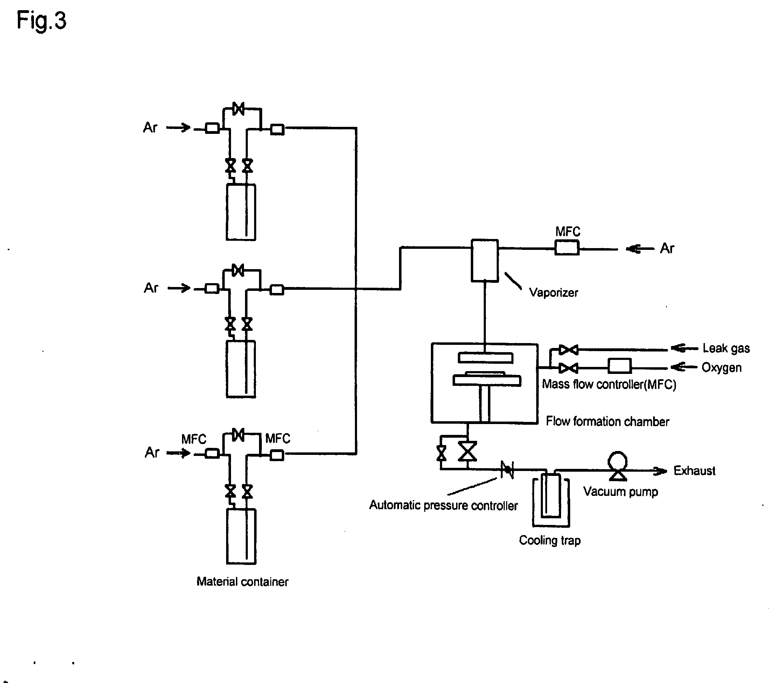

Compound No. 1, tetrakis(1-methoxy-2-methyl-2-propoxy)titanium, and tetrakis(1-methoxy-2-methyl-2-propoxy)zirconium were separately dissolved in ethylcyclohexane having been dried to a water content less than 1 ppm to a concentration of 0.2 mol/l, 0.1 mol/l, and 0.1 mol/l, respectively, to prepare materials for CVD. A PZT thin film was formed on a silicon wafer using the resulting materials by the use of the CVD system shown in Fig. 3 under the following conditions and in accordance with the following steps. The thickness and the composition of the resulting thin film were measured by X-ray fluorescence analysis. The results are shown below.

### Film formation conditions:

Lead source: 0.2 mol/l solution of compound No. 1 in ethylcyclohexane (material flow rate: 0.5 sccm)
Titanium source: 0.1 mol/l solution of tetrakis(1-methoxy-2-methyl-2-propoxy)titanium (material flow rate: 0.55 sccm)
Zirconium source: 0.1 mol/l solution of tetrakis(1-methoxy-2-methyl-2-propoxy)zirconium (material flow rate: 0.45 sccm)
Vaporizer temperature: 180°C
Oxidizing gas: oxygen 200 sccm
Reaction pressure: 1300 to 1400 Pa
Reaction temperature (substrate temperature): 480°C
Film forming time: 10 min
Annealing: 480°C, 3 mins in oxygen

### Results:

Film thickness: 80 nm
Composition (by mole): Pb/Ti/Zi=1.00/0.56/0.47

### EXAMPLE 8: Formation of PZT thin film

To 1000 ml of ethylcyclohexane dehydrated to a water content less than 1 ppm were added 0.1 mol of compound No. 1, 0.055 mol of tetrakis(1-methoxy-2-methyl-2-propoxy)titanium, and 0.045 mol of tetrakis(1-methoxy-2-methyl-2-propoxy)zirconium to prepare a material for CVD. A thin film was formed using the CVD system shown in Fig. 2 and the resulting material for CVD under the following conditions. The thickness and composition of the resulting film were measured in the same manner as in Example 4. The results of measurement are shown below.

### Film forming conditions:

Material flow rate: 1.5 sccm
Vaporizer temperature: 180°C
Oxidizing gas: oxygen 200 sccm
Reaction pressure: 1300 to 1400 Pa
Reaction temperature (substrate temperature): 480°C
Film forming time: 8 min
Annealing: 480°C, 3 min in oxygen

### Results:

Thickness: 50 nm
Composition (by mole): Pb/Ti/Zr=1.00/0.57/0.48

### Industrial Applicability:

The present invention provides a lead compound, a titanium compound, and a zirconium compound each of which has a low melting point and is therefore deliverable in a liquid state, has a high vapor pressure and is therefore easy to vaporize, and, when mixed with other metal compound, undergoes no denaturation due to a chemical reaction. By the use of these compounds, the invention provides a material for thin film formation suitable in thin film forming processes involving a vaporization step, such as CVD, and a CVD process of thin film formation.

## Claims

1. A metal compound represented by general formula (I): 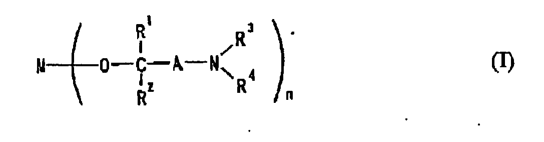 wherein R¹, R², R³, and R⁴ each represent a methyl group; A represents a methylene group; M represents a lead atom, a titanium atom or a zirconium atom; n represents 2 when M is a lead atom or 4 when M is a titanium or zirconium atom.

2. The metal compound according to claim 1, wherein M is a lead atom.

3. The metal compound according to claim 1, wherein M is a titanium atom.

4. The metal compound according to claim 1, wherein M is a zirconium atom.

5. A material for thin film formation comprising the metal compound according to any one of claims 1 to 4.

6. A material for thin film formation comprising the metal compound of claim 2, the metal compound of claim 3, and the metal compound of claim 4.

7. A material for thin film formation comprising the metal compound of claim 2, tetrakis(1-methoxy-2-methyl-2-propoxy)titanium, and tetrakis(1-methoxy-2-methyl-2-propoxy)zirconium.

8. A process for thin film formation comprising vaporizing the material for thin film formation according to claim 5, 6 or 7, introducing the resulting vapor containing the metal compound onto a substrate, and causing the vapor to decompose and/or chemically react to form a metal-containing thin film on the substrate.

9. A process for thin film formation comprising vaporizing a material for thin film formation containing the metal compound of claim 2, a material for thin film formation containing the metal compound of claim 3, and a material for thin film formation containing the metal compound of claim 4 to obtain vapor containing the metal compounds, introducing the resulting vapor containing the metal compounds onto a substrate, and causing the vapor to decompose and/or chemically react to form a metal-containing thin film on the substrate.

10. A process for thin film formation comprising vaporizing a material for thin film formation containing the metal compound of claim 2, a material for thin film formation containing tetrakis(1-methoxy-2-methyl-2-propoxy)titanium, and a material for thin film formation containing tetrakis(1-methoxy-2-methyl-2-propoxy)zirconium to obtain vapor containing the metal compounds, introducing the resulting vapor containing the metal compounds onto a substrate, and causing the vapor to decompose and/or chemically react to form a metal-containing thin film on the substrate.

11. A process for thin film formation comprising vaporizing a material for thin film formation containing the metal compound
of claim 2, a material for thin film formation containing tetra(tert-butoxy)titanium, and a material for thin film formation containing tetra(tert-butoxy)zirconium to obtain vapor containing the metal compounds, introducing the resulting vapor containing the metal compounds onto a substrate, and causing the vapor to decompose and/or chemically react to form a metal-containing thin film on the substrate.

## Patentansprüche

1. Metallverbindung, dargestellt durch die allgemeine Formel (I): 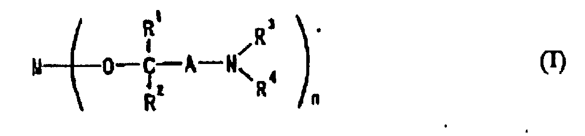 wobei R¹, R², R³ und R⁴ jeweils eine Methylgruppe darstellt; A eine Methylengruppe darstellt; M ein Bleiatom, ein Titanatom oder ein Zirkoniumatom darstellt; n 2 darstellt, wenn M ein Bleiatom ist, oder 4, wenn M ein Titan- oder Zirkoniumatom ist.

2. Die Metallverbindung nach Anspruch 1, wobei M ein Bleiatom ist.

3. Die Metallverbindung nach Anspruch 1, wobei M ein Titanatom ist.

4. Die Metallverbindung nach Anspruch 1, wobei M ein Zirkoniumatom ist.

5. Ein Material zur Bildung eines dünnen Films, umfassend die Metallverbindung nach irgendeinem der Ansprüche 1 bis 4.

6. Ein Material zur Bildung eines dünnen Films, umfassend die Metallverbindung von Anspruch 2, die Metallverbindung von Anspruch 3, und die Metallverbindung von Anspruch 4.

7. Ein Material zur Bildung eines dünnen Films, umfassend die Metallverbindung von Anspruch 2, Tetrakis(1-methoxy-2-methyl-2-propoxy)titan, und Tetrakis(1-methoxy-2-methyl-2-propoxy)zirkonium.

8. Verfahren zur Bildung eines dünnen Films, umfassend das Verdampfen des Materials zur Bildung eines dünnen Films gemäß Anspruch 5, 6 oder 7, zuführen des resultierenden Dampfes, der die Metallverbindung enthält, zu einem Substrat, und bewirken, dass der Dampf sich zersetzt und/oder chemisch reagiert, um einen Metall-enthaltenden dünnen Film auf dem Substrat zu bilden.

9. Verfahren zur Bildung eines dünnen Films, umfassend das Verdampfen eines Materials zur Bildung eines dünnen Films, enthaltend die Metallverbindung von Anspruch 2, ein Material zur Bildung eines dünnen Films, enthaltend die Metallverbindung von Anspruch 3, und ein Material zur Bildung eines dünnen Films, enthaltend die Metallverbindung von Anspruch 4, um Dampf zu erhalten, der die Metallverbindungen enthält, zuführen des resultierenden Dampfes, der die Metallverbindungen enthält, zu einem Substrat, und bewirken, dass der Dampf sich zersetzt und/oder chemisch reagiert, um einen Metall-enthaltenden dünnen Film auf dem Substrat zu bilden.

10. Verfahren zur Bildung eines dünnen Films, umfassend das Verdampfen eines Materials zur Bildung eines dünnen Films, enthaltend die Metallverbindung von Anspruch 2, ein Material zur Bildung eines dünnen Films, enthaltend Tetrakis(1-methoxy-2-methyl-2-propoxy)titan, und ein Material zur Bildung eines dünnen Films, enthaltend Tetrakis(1-methoxy-2-methyl-2-propoxy)zirkonium, um Dampf zu erhalten, der die Metallverbindungen enthält, zuführen des resultierenden Dampfes, der die Metallverbindungen enthält, zu einem Substrat, und bewirken, dass der Dampf sich zersetzt und/oder chemisch reagiert, um einen Metall-enthaltenden dünnen Film auf dem Substrat zu bilden.

11. Verfahren zur Bildung eines dünnen Films, umfassend das Verdampfen eines Materials zur Bildung eines dünnen Films, enthaltend die Metallverbindung von Anspruch 2, ein Material zur Bildung eines dünnen Films, enthaltend Tetra(tert-butoxy)titan, und ein Material zur Bildung eines dünnen Films, enthaltend Tetra(tert-butoxy)zirkonium, um Dampf zu erhalten, der die Metallverbindungen enthält, zuführen des resultierenden Dampfes, der die Metallverbindungen enthält, zu einem Substrat, und bewirken, dass der Dampf sich zersetzt und/oder chemisch reagiert, um einen Metall-enthaltenden dünnen Film auf dem Substrat zu bilden.

## Revendications

1. Composé métallique représenté par la formule générale (I): 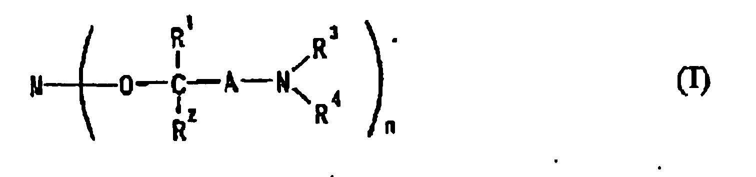 où R¹, R², R³ et R⁴ représentent chacun un groupement méthyle ; A représente un groupement méthylène ; M représente un atome de plomb, un atome de titane ou un atome de zirconium ; n représente 2 lorsque M est un atome de plomb ou 4 lorsque M est un atome de titane ou de zirconium.

2. Composé métallique selon la revendication 1, dans laquelle M est un atome de plomb.

3. Composé métallique selon la revendication 1, où M est un atome de titane.

4. Composé métallique selon la revendication 1, où M est un atome de zirconium.

5. Matériau destiné à la formation de film mince comprenant le composé métallique selon l'une quelconque des revendications 1 à 4.

6. Matériau destiné à la formation de film mince comprenant le composé métallique de la revendication 2, le composé métallique de la revendication 3, et le composé métallique de la revendication 4.

7. Matériau destiné à la formation de film mince comprenant le composé métallique de la revendication 2, le tétrakis(1-méthoxy-2-méthyl-2-propoxy)titane, et le tétrakis(1-méthoxy-2-méthyl-2-propoxy)zirconium.

8. Processus destiné à la formation de film mince comprenant le fait de vaporiser le matériau pour la formation de film mince selon la revendication 5, 6 ou 7, d'introduire la vapeur résultante contenant le composé métallique sur un substrat, et d'amener la vapeur à se décomposer et/ou à réagir chimiquement pour former un film mince contenant du métal sur le substrat.

9. Processus destiné à la formation de film mince comprenant le fait de vaporiser un matériau pour la formation de film mince contenant le composé métallique de la revendication 2, un matériau pour la formation de film mince contenant le composé métallique de la revendication 3, et un matériau pour la formation de film mince contenant le composé métallique de la revendication 4 pour obtenir de la vapeur contenant les composés métalliques, d'introduire la vapeur résultante contenant les composés métalliques sur un substrat, et d'amener la vapeur à se décomposer et/ou à réagir chimiquement pour former un film mince contenant du métal sur le substrat.

10. Processus destiné à la formation de film mince comprenant le fait de vaporiser un matériau pour la formation de film mince contenant le composé métallique de la revendication 2, un matériau pour la formation de film mince contenant le tétrakis(1-méthoxy-2-méthyl-2-propoxy)titane, et un matériau pour la formation de film mince contenant le tétrakis(1-méthoxy-2-méthyl-2-propoxy)zirconium pour obtenir de la vapeur contenant les composés métalliques, d'introduire la vapeur résultante contenant les composés métalliques sur un substrat, et d'amener la vapeur à se décomposer et/ou à réagir chimiquement pour former un film mince contenant du métal sur le substrat.

11. Processus destiné à la formation de film mince comprenant le fait de vaporiser un matériau pour la formation de film mince contenant le composé métallique de la revendication 2, un matériau pour la formation de film mince contenant le tétra(tert-butoxy)titane, et un matériau pour la formation de film mince contenant le tétra(tert-butoxy)zirconium pour obtenir de la vapeur contenant les composés métalliques, d'introduire la vapeur résultante contenant les composés métalliques sur un substrat, et d'amener la vapeur à se décomposer et/ou à réagir chimiquement pour former un film mince contenant du métal sur le substrat.
